## Europäisches Patentamt
## European Patent Office
## Office européen des brevets

(19)

(11) Veröffentlichungsnummer: **0 228 623**
**B1**

(12)

# EUROPÄISCHE PATENTSCHRIFT

(45) Veröffentlichungstag der Patentschrift:
**15.02.89**

(21) Anmeldenummer: **86117160.1**

(22) Anmeldetag: **09.12.86**

(51) Int. Cl.⁴: **C 07 C 7/09**, C 07 C 7/04,
F 25 J 3/00

(54) Verfahren zur Abtrennung von C5+-Kohlenwasserstoffen aus einem Gasstrom.

(30) Priorität: **18.12.85 DE 3544855**

(43) Veröffentlichungstag der Anmeldung:
**15.07.87 Patentblatt 87/29**

(45) Bekanntmachung des Hinweises auf die Patenterteilung:
**15.02.89 Patentblatt 89/7**

(84) Benannte Vertragsstaaten:
**CH DE FR GB IT LI NL SE**

(56) Entgegenhaltungen:
**EP-A-0 185 253**
**DD-A-204 471**

(73) Patentinhaber: **Linde Aktiengesellschaft, Abraham-Lincoln- Strasse 21, D-6200 Wiesbaden (DE)**

(72) Erfinder: **Heinz, Bauer, Dr.rer.nat.,
Nibelungenstrasse 6, D-8000 München 19 (DE)**

(74) Vertreter: **Schaefer, Gerhard, Dr., Linde Aktiengesellschaft Zentrale Patentabteilung, D-8023 Höllriegelskreuth (DE)**

## Beschreibung

Die Erfindung betrifft ein Verfahren zur Abtrennung von $C_{5+}$-Kohlenwasserstoffen aus einem leichte Kohlenwasserstoffe und gegebenenfalls leichter als Methan siedende Komponenten enthaltenden Gasstrom, bei dem der unter erhöhtem Druck stehende Gasstrom abgekühlt, partiell kondensiert und in eine flüssige und eine gasförmige Fraktion getrennt wird und bei dem die flüssige Fraktion durch Rektifikation in einen im wesentlichen $C_{5+}$-Kohlenwasserstoffe enthaltenden Produktstrom und einen überwiegend leichter siedende Komponenten enthaltenden Restgasstrom zerlegt wird.

In der DE-A-3 511 636 wird ein Verfahren zur Abtrennung von $C_{2+}$- oder von $C_{3+}$-Kohlenwasserstoffen aus einem Gasstrom vorgeschlagen, bei dem in einer Ausführungsform vor der Abtrennung dieser Kohlenwasserstoffe noch eine separate Abtrennung einer an $C_{5+}$-Kohlenwasserstoffen angereicherten Fraktion erfolgt. Die an $C_{5+}$-Kohlenwasserwasserstoffen angereicherte Fraktion wird dabei einer Rektifiziersäule zugeführt, in der auch die $C_{2+}$- bzw. $C_{3+}$-Kohlenwasserstoffe gereinigt und als Produktströme über einen Seitenanstich aus der Rektifiziersäule abgezogen werden, während eine $C_{5+}$-Kohlenwasserstoffe enthaltende Fraktion aus dem Säulensumpf abgezogen wird.

Verfahren, bei denen die $C_2$- bis $C_4$- oder $C_3/C_4$-Kohlen-wasserstoffgemische (LPG) als Seitenprodukt einer Kolonne mit großem Trennbereich gewonnen werden, sind jedoch kostenmäßig ungünstig, da selbst bei großen Bodenzahlen in der Rektifiziersäule große interne Umsätze zur Reindarstellung des Seitenprodukts benötigt werden. Die bei einer Rektifiziersäule mit $C_2$-Kohlenwasserstoffen als Kopfprodukt und $C_{5+}$-Kohlenwasserstoffen als Sumpfprodukt zwangsläufig zwischen Säulenkopf und Säulensumpf auftretende große Temperaturdifferenz, die beispielsweise in der Größenordnung von etwa 200°C liegt, verursacht wegen der damit verbundenen Irreversibilitäten einen hohen spezifischen Energiebedarf. Die $C_4/C_5$-Trennung im unteren Abschnitt einer derartigen Rektifiziersäule muß dabei in der Regel unter einem für diese spezielle Trennaufgabe ungünstig hohen Druck erfolgen, um gute Kondensationsbedingungen für das Kopfprodukt zu schaffen. Eine alternative Möglichkeit der Abtrennung von $C_{5+}$-Kohlenwasserstoffen aus einem derartigen Gasgemisch besteht darin, daß zunächst sämtliche $C_{2+}$- bzw. $C_{3+}$-Kohlenwasserstoffe abgetrennt und aus dem dabei gewonnenen Produktstrom dann in einem zusätzlichen Debutanizer die $C_{5+}$-Kohlenwasser-stoffe abgetrennt werden. Diese Verfahrensweise ist jedoch auch ungünstig, da ein zusätzlicher Debutanizer benötigt wird, was beträchtliche Investitions- und Betriebskosten verursacht.

Eine einfache Vorabtrennung der $C_{5+}$-Kohlenwasserstoffe durch partielle Kondensation ist in der Regel auch nicht zufriedenstellend, da wegen der kleinen Flüchtigkeitsunterschiede zwischen Butan und Pentan eine schlechte Selektivität zur $C_{5+}$-Abtrennung vorliegt. Bei einer ausreichenden $C_{5+}$-Abtrennung wird somit unerwünscht viel $C_4$ mitkondensiert was in vielen Fällen unerwünscht ist, da LPG oft als besonders erwünschtes Verfahrensprodukt erzeugt werden soll.

Der Erfindung liegt die Aufgabe zugrunde, ein Verfahren der eingangs genannten Art so auszugestalten, daß es ohne hohen Aufwand an Investitions- und Betriebskosten möglich. ist, die $C_{5+}$-Kohlenwasserstoffe aus dem Gasstrom abzutrennen. Insbesondere soll das erfindungsgemäße Verfahren auch in der Lage sein, aus einem $C_{5+}$-Kohlenwasserstoffe und LPG sowie leichtere Komponenten enthaltenden Gas sowohl die $C_{5+}$-Kohlenwasserstoffe als auch LPG in hoher Ausbeute abzutrennen.

Diese Aufgaben werden dadurch gelöst, daß bei einem Verfahren der eingangs genannten Art die nach der partiellen Kondensation abgetrennte flüssige Fraktion vor der Rektifikation auf einen tieferen Druck entspannt und die nach der partiellen Kondensation abgetrennte gasförmige Fraktion einer Rückwaschkolonne zugeführt wird, in der mit bei der Rektifikation gewonnenem Restgas nach dessen teilweiser Kondensation $C_{5+}$-Kohlenwasserstoffe aus der gasförmigen Fraktion ausgewaschen werden und die im Sumpf der Rückwaschkolonne anfallende flüssige Fraktion nach Entspannung der Rektifikation zugeführt wird.

Das erfindungsgemäße Verfahren geht aus von der Grundüberlegung, daß die $C_{5+}$-Kohlenwasserstoffe bei einer relativ hohen Temperatur abgetrennt und nicht unnötig unterkühlt werden sollen. Bei der gleichzeitigen Gewinnung von $C_{5+}$-Kohlenwasserstoffen und LPG von leichteren Komponenten soll dabei die Abtrennung in zwei separaten Schritten erfolgen. Da eine ausreichende $C_{5+}$-Abtrennung allein durch partielle Kondensation nicht erreicht werden kann und bei zusätzlicher LPG-Gewinnung ein Großteil der $C_3$-und insbesondere der $C_4$-Kohlenwasserstoffe gemeinsam mit den $C_{5+}$-Kohlenwasserstoffen kondensieren würde, wird erfindungsgemäß eine zusätzliche selbstkühlende Wäsche in einer Rückwaschkolonne durchgeführt, bei der nach der partiellen Kondensation in der Gasphase verbliebene schwerere Komponenten mit einer leichteren Waschflüssigkeit, die während der Wäsche teilweise verdampft, abgetrennt werden. Als Waschflüssigkeit wird dabei eine an $C_{5+}$-Kohlenwasserstoffen freie Fraktion, nämlich das bei der nachfolgenden Rektifikation des Kondensats anfallende Kopfprodukt verwendet. Die Rektifikation ist ausschließlich auf die Gewinnung einer $C_{5+}$-Kohlenwasserstoff-Fraktion ausgerichtet und kann daher unter günstigen Bedingungen für die Trennung eines $C_4/C_5$-

Schnitts durchgeführt werden, insbesondere bei einem relativ niedrigen Druck.

Die im Sumpf der Rückwaschkolonne anfallende flüssige Fraktion enthält zwar die aus der gasförmigen Fraktion ausgewaschenen schweren Bestandteile, doch liegt die Konzentration dieser Komponenten deutlich unter derjenigen in der partiell kondensierten flüssigen Fraktion. In einer günstigen Weiterbildung der Erfindung ist deshalb vorgesehen, daß die im Sumpf der Rückwaschkolonne anfallende flüssige Fraktion der Rektifikation separat aufgegeben wird, vorzugsweise als Rücklaufflüssigkeit.

Beim erfindungsgemäßen Verfahren erfolgt die partielle Kondensation zur Abtrennung der $C_{5+}$ - angereicherten Fraktion üblicherweise in einem Temperaturbereich zwischen +10 und -15°C.

Die Rektifikation wird in vorteilhafter Weise so ausgeführt, daß der am Kopf der Rektifiziersäule anfallende Restgasstrom keine $C_{5+}$-Kohlenwasserstoffe mehr enthält, während häufig ein geringer Anteil an $C_3$- und $C_4$-Kohlenwasserstoffen im Sumpfprodukt zugelassen werden kann. Die Rektifizierbedingungen können daher so optimiert werden, daß der Rektifizieraufwand einerseits möglichst gering ausfällt und andererseits bei gleichzeitiger Gewinnung einer LPG-Fraktion auch nur so wenig $C_3$- und $C_4$-Kohlenwasserstoffe im Sumpfprodukt der Rektifikation abgezogen werden, daß die im Gasstrom verbleibenden $C_3$-und $C_4$-Kohlenwasserstoffe noch zu einer ausreichenden LPG-Ausbeute des Verfahrens führen. Ein geringer Restgehalt an $C_3$- und $C_4$-Kohlenwasserstoffen im $C_{5+}$-Produkt-strom der Rektifikation kann in vielen Fällen in ohnehin vorhandenen nachgeschalteten Anlagen abgetrennt werden. So ist beispielsweise ein typischer Anwendungsfall für das erfindungsgemäße Verfahren die Aufbereitung eines Raffineriegases, die innerhalb einer großen Gesamtanlage erfolgt, in der ohnehin Trennsäulen für eine $C_4/C_5$-Trennung vorgesehen sind. Sofern das $C_{5+}$-Sumpfprodukt der Rektifikation nur noch relativ geringe Mengen leichterer Komponenten enthält, können diese häufig ohne großen zusätzlichen Aufwand in derartige vorhandene Trenneinrichtungen zusätzlich eingeführt werden.

Das bei der Rektifikation anfallende Kopfprodukt muß vor seiner Verwendung als Waschflüssigkeit in der Rückwaschkolonne wieder auf den erhöhten Druck des zu zerlegenden Gasstroms rückverdichtet werden. Dies erfolgt vorteilhaft dadurch, daß das Restgas der Rektifikation zunächst teilweise kondensiert wird, beispielsweise zu 50 bis 99 %, vorzugsweise zu 70 bis 95 %, und daß der dabei gewonnene flüssige Anteil abgetrennt und separat auf höheren Druck gepumpt wird, um schließlich der Rückwaschkolonne zugeführt zu werden. Der nicht kondensierte Anteil des Restgases der Rektifikation kann dann beim niedrigeren Druck ohne Verdichtung als Restgasstrom abgegeben werden. Die partielle Kondensation des Restgases der Rektifikation kann in vorteilhafter Weise durch Abkühlung auf Temperaturen zwischen -25 und -50 °C erfolgen.

Die bei der partiellen Kondensation des zu zerlegenden Gasstroms gewonnene flüssige Fraktion wird in günstiger Weiterbildung der Erfindung vor der Rektifikation mindestens teilweise gegen den abzukühlenden Gasstrom erwärmt, um günstige Rektifikationsbedingungen einzustellen.

Sofern neben der Gewinnung der $C_{5+}$-Fraktion auch eine LPG-Fraktion als Verfahrensprodukt gewonnen werden soll, wird in Weiterbildung der Erfindung die aus der Rückwaschkolonne austretende gasförmige Fraktion durch weitere Abkühlung und partielle Kondensation in eine weitere flüssige und gasförmige Fraktion getrennt und die dabei gewonnene weitere flüssige Fraktion durch eine weitere Rektifikation in einen im wesentlichen $C_{3+}$-Kohlenwasserstoffe enthaltenden weiteren Produktstrom und einen überwiegend leichter siedende Komponenten enthaltenden weiteren Restgasstrom zerlegt. Sollte statt einer LPG-Gewinnung die Gewinnung einer $C_2$- bis $C_4$-Fraktion beabsichtigt sein, kann in völlig analoger Weise vorgegangen werden. In günstiger Weiterbildung dieses Verfahrens wird der weitere Restgasstrom zur Kälteleistung mindestens teilweise arbeitsleistend entspannt und der Druck der ersten Rektifikation zur Abtrennung der $C_{5+}$-Kohlen-wasserstoffe so eingestellt, daß er zwischen dem erhöhten Druck des zu zerlegenden Gasstromes und dem Druck des arbeitsleistend entspannten weiteren Restgasstroms liegt. Der Druck der ersten Rektifikation liegt dabei insbesondere so nahe am Druck des weiteren Restgasstroms, daß die bei der partiellen Kondensation des ersten Restgases nicht kondensierten Anteile direkt in den arbeitsleistend entspannten Teil des weiteren Restgases eingeführt werden können.Auf diese Weise erübrigt sich eine separate Abführung von zwei Restgasströmen.

Eine Anlage zur Durchführung des erfindungsgemäßen Verfahrens enthält mindestens einen Wärmetauscher zur Abkühlung und partiellen Kondensation des Gasstroms, einen Abscheider zur Abtrennung des partiell kondensierten Teils des Gasstroms und eine Rektifiziersäule zur Zerlegung des partiell kondensierten Teils des Gasstroms sowie eine Rückwaschkolonne, deren unterer Bereich mit dem Dampfraum des Abscheiders verbunden und deren oberer Bereich mit dem Kopf der Rektifiziersäule verbunden ist, wobei zwischen dem Kopf der Rektifiziersäule und dem oberen Bereich der Rückwaschkolonne ein Wärmetauscher, ein weiterer Abscheider und eine Pumpe geschaltet sind. In weiterer Ausbildung der Anlage sind der Abscheider und die Rückwaschkolonne in einem gemeinsamen Behälter angeordnet, wobei die Rückwaschkolonne vorzugsweise oberhalb des Abscheiders angeordnet ist und durch einen Kaminboden vom Abscheider getrennt ist.

Weitere Einzelheiten der Erfindung werden nachfolgend anhand der Figuren erläutert.

Es zeigen:

Figur 1    eine schematische Darstellung eines Ausführungsbeispiels der Erfindung und

Figur 2    die Temperaturabhängigkeit von $C_4/C_{5+}$-Kohlenwasser-stoffen bei der partiellen Kondensation des Einsatzgasgemisches.

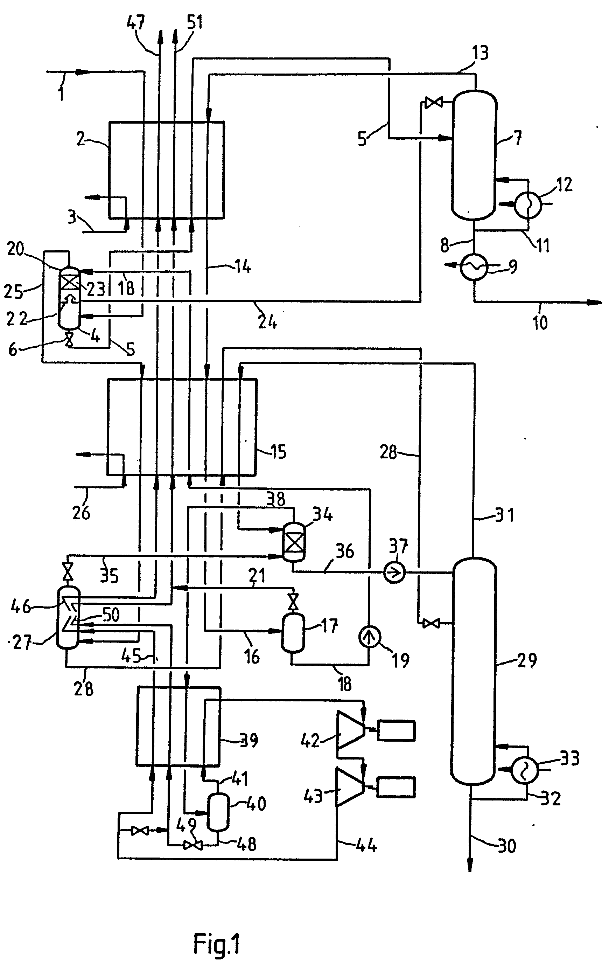

Bei der in Figur 1 schematisch dargestellten Anlage wird über Leitung 1 das zu zerlegende Gasgemisch, beispielsweise ein Raffineriegas oder Erdgas, bei erhöhtem Druck, beispielsweise zwischen 15 und 25 bar, und bei annähernd Umgebungstemperatur einem Wärmetauscher 2 zugeführt, in dem es gegen anzuwärmende Verfahrensströme sowie gegen einen Kühlkreislauf 3 so weit abgekühlt wird, daß der größte Teil der $C_{5+}$-Kohlenwasserstoffe sowie im Gleichgewicht damit ein Teil der $C_3$- und $C_4$-Kohlenwasserstoffe kondensiert. Im Abscheider 4 wird das gebildete Kondensat von der gasförmig verbliebenen Fraktion abgetrennt, über Leitung 5 abgezogen, im Ventil 6 auf einen niedrigeren Druck entspannt und im Wärmetauscher 2 wieder angewärmt, bevor es nach Teilverdampfung in eine Rektifiziersäule 7 eingespeist wird. Im Sumpf der Rektifiziersäule 7 fällt über Leitung 8 ein Produktstrom an, der im wesentlichen $C_{5+}$-Kohlenwasserstoffe und daneben noch geringe Mengen an $C_3$- und $C_4$-Kohlenwasserstoffen enthält. Er wird nach Abkühlung im Wärmetauscher 9 über Leitung 10 als Produktstrom abgeführt und kann beispielsweise einem im Rahmen einer größeren Anlage ohnehin vorhandenen Debutanizer zugeführt werden, in dem es ohne großen Aufwand weiter in eine $C_3/C_4$-Fraktion und eine $C_{5+}$-Fraktion zerlegt werden kann. Ein Teil der aus dem Sumpf der Rektifiziersäule 7 abgezogenen Flüssigkeit wird über Leitung 11 einem Wärmetauscher 12 zugeführt, darin erwärmt und zur Sumpfbeheizung der Rektifiziersäule 7 wieder in den unteren Bereich der Säule zurückgeführt. Am Kopf der Rektifiziersäule 7 fällt über Leitung 13 ein leichtes Produkt an, das keine $C_{5+}$-Kohlenwasserstoffe mehr enthält. Es wird im Wärmetauscher 2 abgekühlt und gelangt danach über Leitung 14 in einen weiteren Wärmetauscher 15, in dem das Kopfprodukt zum größeren Teil kondensiert wird. Das Kondensat gelangt über Leitung 16 in einen Abscheider 17, in dem die gebildete Flüssigkeit abgetrennt und über Leitung 18 abgezogen wird. Nach Druckerhöhung auf den Druck des Rohgases mittels der Pumpe 19 wird die Flüssigkeit durch den Wärmetauscher 15 geführt und danach auf den Kopf einer Rückwaschsäule 20 gegeben. Der im Abscheider 17 anfallende Restgasstrom wird über Leitung 21 abgezogen und in eine weiter unten noch zu beschreibende Restgasleitung eingespeist.

Die Rückwaschsäule 20 ist oberhalb des Abscheiders 4 angeordnet und durch einen Kaminboden 22 von ihm getrennt. Durch den Kaminboden tritt die gasförmige Fraktion aus dem Abscheider 4 in den unteren Bereich der Rückwaschkolonne ein und wird bei ihrer aufwärts gerichteten Strömung innerhalb einer Schüttung 23 oder einiger Böden in Kontakt mit dem über Leitung 18 herangeführten Kondensat gebracht. Dabei werden noch im Gas verbliebene höhere Komponenten ausgewaschen, während ein Teil der Waschflüssigkeit verdampft und in der gasförmigen Fraktion verbleibt. Im unteren Bereich der Rückwaschkolonne fällt eine mit schweren Komponenten beladene Flüssigkeit an die über Leitung 24 abgezogen und als Rücklaufflüssigkeit auf den Kopf der Rektifiziersäule 7 gegeben wird.

Die aus der Rückwaschsäule 20 austretende gasförmige Fraktion wird über Leitung 25 abgezogen und im Wärmetauscher 15 gegen anzuwärmende Verfahrensströme sowie gegen weitere Fremdkälte in einem Kühlkreislauf 26 erneut partiell kondensiert, um $C_3$- und $C_4$-Kohlenwasserstoffe weitgehend abzutrennen. Das abgekühlte Gemisch wird in einen Abscheider 27 geleitet. Kondensat gelangt über Leitung 28 nach erneuter Anwärmung im Wärmetauscher 15 in eine weitere Rektifiziersäule 29 und wird dort in eine im Sumpf anfallende und über Leitung 30 abgezogene LPG-Fraktion sowie in ein leichter siedende Komponenten enthaltendes, über Leitung 31 abgezogenes Kopfprodukt zerlegt. Ein Teil des im Sumpf anfallenden LPG wird über Leitung 32 abgezweigt, im Wärmetauscher 33 erwärmt und zur Sumpfbeheizung in den unteren Bereich der Säule 29 zurückgeführt. Das über Leitung 31 aus der Säule 29 abgezogene Kopfprodukt wird im Wärmetauscher 15 soweit wieder abgekühlt, daß es partiell kondensiert und dann in eine weitere Rückwaschsäule 34 geführt. Der kondensierte Anteil des Kopfprodukts wäscht hier die aus dem Abscheider 27 über Leitung 35 abgezogene gasförmige Fraktion, wobei in dieser Fraktion verbliebene $C_3$- und $C_4$-Kohlenwasserstoffe im wesentlichen beim Druck der Rektifiziersäule 29 ausgewaschen werden. Die dabei anfallende flüssige Fraktion wird über Leitung 36 abgezogen und mittels der Pumpe 37 als Rücklaufflüssigkeit auf den Kopf der Rektifiziersäule 29 gefördert. Die aus der Rückwaschsäule 34 austretende gasförmige Fraktion gelangt über Leitung 38 in einen weiteren Wärmetauscher 39 und wird dort soweit abgekühlt, daß durch partielle Kondensation und Abtrennung des Kondensats in einem Abscheider 40 eine im wesentlichen Wasserstoff enthaltende gasförmige Fraktion und eine höhersiedende Komponenten enthaltende flüssige Fraktion gebildet werden. Die Wasserstoff-Fraktion wird über Leitung 41 abgezogen, im Wärmetauscher 39 teilweise angewärmt und dann in einer Expansionsturbine 42, 43 zweistufig arbeitsleistend entspannt. Der entspannte Wasserstoff gelangt über Leitung 44 ans kalte Ende des Wärmetauschers 39 und wird im Wärmetausch mit dem über Leitung 38 herangeführten Gasstrom angewärmt. Anschließend gelangt der Wasserstoff in einen Wärmetauscher 46, der innerhalb des Abscheiders 27 angeordnet ist und hier die gasförmige Fraktion weiter kühlt, um dadurch einen Teil der im Gas verbliebenen $C_3$- und $C_4$-Kohlenwasserstoffe abzutrennen. Danach wird der Wasserstoff in den Wärmetauschern 15 und 2 weiter angewärmt und verläßt über Leitung 47 die Anlage als Produktstrom. Die im Abscheider 40 anfallende flüssige Fraktion wird über Leitung 48 abgezogen, im Ventil 49 entspannt und danach im Wärmetauscher 39 angewärmt. Auch diese Fraktion wird anschließend im Abscheider 27 weiter gegen die dort vorliegende gasförmige Fraktion angewärmt, wozu der Wärmetauscher 50 vorgesehen ist, und dann nach Vermischung mit dem über Leitung 21 aus dem Abscheider 17 herangeführten Restgas in den Wärmetauschern 15 und 2 angewärmt, um schließlich über Leitung 51 als Restgasfraktion abgegeben zu werden.

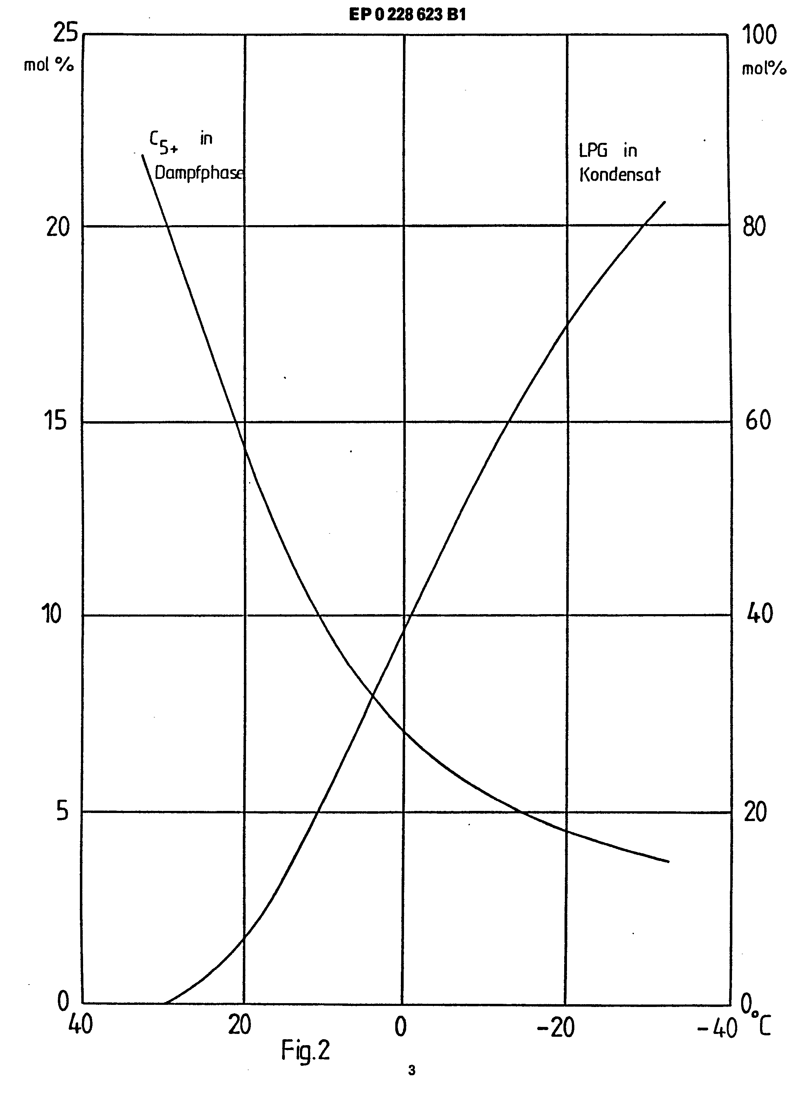

In der Figur 2 ist die Zusammensetzung der im Abscheider 4 anfallenden Fraktionen in Abhängigkeit von der Temperatur aufgetragen. Um eine gute Abtrennung von $C_{5+}$-Kohlenwasserstoffen zu erreichen, die eine nachfolgende Behandlung des in der Säule 29 anfallenden LPG-Produkts erübrigt, ist typischerweise ein Restgehalt an $C_{5+}$-Kohlenwasserstoffen von weniger als 3 %, beispielsweise von 2 %, in der weiter zu zerlegenden gasförmigen Fraktion erforderlich. Aus der Figur 2 ist ersichtlich, daß dies durch partielle Kondensation nur dann erreicht werden kann, wenn das Gas vor der Phasentrennung schon auf sehr tiefe Temperaturen abgekühlt worden ist. Dies hat jedoch den nachteiligen Effekt, daß dann auch schon ein sehr großer Anteil der $C_3$/$C_4$-Komponenten mit kondensiert, so daß das dann in der Rektifiziersäule 29 noch in ausreichender Reinheit zu gewinnende LPG-Produkt nur noch in kleiner Menge anfällt. Beispielsweise ergibt sich bei einer typischen Abscheidetemperatur von +3° C ein unlässig hoher $C_{5+}$-Kohlenwasserstoffgehalt in der Gasphase von etwa 8 Mol-%, während in der flüssigen Phase bereits etwa 32 Mol-% des LPG kondensiert und für die nachfolgende Zerlegungsstufe verloren sind. Wegen des hohen LPG-Anteils dieser Fraktion ist auch eine Weiterverarbeitung in üblichen, in Raffinerien häufig vorhandenen Trennkolonnen nur mit großem Aufwand zu verwirklichen. Bei Anwendung des erfindungsgemäßen Verfahrens ergibt sich bei dem gleichen Gasgemisch in Leitung 25 jedoch eine Gaszusammensetzung mit nur etwa 2,3 Mol-% $C_{5+}$-Kohlenwasser-stoffe, während gleichzeitig die in Leitung 10 gewonnene $C_{5+}$-Fraktion einen LPG-Anteil von nur noch etwa 22 % enthält, so daß etwa 78 % des im Rohgas enthaltenen LPG über Leitung 30 als Produktstrom in spezifikationsgerechter Reinheit abgezogen werden können.

**Patentansprüche**

1. Verfahren zur Abtrennung von $C_{5+}$-Kohlenwasserstoffen aus einem leichte Kohlenwasserstoffe und gegebenenfalls leichter als Methan siedende Komponenten enthaltenden Gasstrom, bei dem der unter erhöhtem Druck stehende Gasstrom (1) abgekühlt, partiell kondensiert (2) und in eine flüssige und in eine gasförmige Fraktion getrennt (4) wird und bei dem die. flüssige Fraktion durch Rektifikation (7) in einen im wesentlichen $C_{5+}$-Kohlenwasserstoffe enthaltenden Produktstrom (8, 10) und einen überwiegend leichter siedende Komponenten enthaltenden Restgasstrom (13) zerlegt wird, dadurch gekennzeichnet, daß die nach der partiellen Kondensation (2) abgetrennte flüssige Fraktion (5) vor der Rektifikation (7) auf einem tieferen Druck entspannt (6) und die nach der partiellen Kondensation abgetrennte gasförmige Fraktion einer Rückwaschkolonne (20) zugeführt wird, in der mit bei der Rektifikation gewonnenem Restgas (13) nach dessen teilweiser Kondensation $C_{5+}$-Kohlenwasserstoffe aus der gasförmigen Fraktion ausgewaschen werden und die im Sumpf der Rückwaschkolonne (20) anfallende flüssige Fraktion (24) nach Entspannung der Rektifiktion (7) zugeführt wird.

2. Verfahren nach Anspruch 1, dadurch gekennzeichnet, daß die im Sumpf der Rückwaschkolonne (20) anfallende flüssige Fraktion (24) der Rektifikation (7) als Rücklaufflüssigkeit aufgegeben wird.

3. Verfahren nach Anspruch 1 oder 2, dadurch gekennzeichnet, daß die durch partielle Kondensation (2) erhaltene flüssige Fraktion (5) bei einer Temperatur zwischen 10 und -15° C abgetrennt (4) wird.

4. Verfahren nach einem der Ansprüche 1 bis 3, dadurch gekennzeichnet, daß die Rektifikation (7) so durchgeführt wird, daß mit dem $C_{5+}$-Produktstrom der Rektifikation (8, 10) ein Teil der $C_3$- und $C_4$-Kohlenwasserstoffe und mit dem Restgasstrom (13) keine $C_{5+}$-Kohlenwasserstoffe abgezogen werden.

5. Verfahren nach einem der Ansprüche 1 bis 4, dadurch gekennzeichnet, daß nach der teilweisen Kondensation (2, 15) des Restgases (13) der Rektifikation (7) eine Phasentrennung erfolgt (17) und die dabei gewonnene flüssige Fraktion (18) auf höheren Druck gepumpt (19) und der Rückwaschkolonne (20) zugeführt wird.

6. Verfahren nach Anspruch 5, dadurch gekennzeichnet, daß der nicht kondensierte Anteil des Restgases der Rektifiktion (21) bei niedrigem Druck als Restgasstrom (21, 51) abgegeben wird.

7. Verfahren nach einem der Ansprüche 1 bis 6, dadurch gekennzeichnet, daß die partielle Kondensation (2, 15) des Restgases (13) der Rektifikation (7) durch Abkühlung auf Temperaturen zwischen -25 und -50°C erfolgt.

8. Verfahren nach einem der Ansprüche 1 bis 7, dadurch gekennzeichnet, daß das bei der Rektifikation (7) gewonnene Restgas (13) vor dessen Einspeisung in die Rückwaschkolonne (20) zu 50 bis 99 %, vorzugsweise zu 70 bis 95 % verflüssigt wird.

9. Verfahren nach einem der Ansprüche 1 bis 8, dadurch gekennzeichnet, daß die flüssige Fraktion (5) vor der Rektifikation (7) mindestens teilweise gegen den abzukühlenden Gasstrom (1) erwärmt wird.

10. Verfahren nach einem der Ansprüche 1 bis 9, dadurch gekennzeichnet, daß die aus der Rückwaschkolonne (20) austretende gasförmige Fraktion (25) durch weitere Abkühlung und partielle Kondensation (15, 39) in eine weitere flüssige (28) und gasförmige (35) Fraktion getrennt (27) und die dabei gewonnene flüssige Fraktion (28) durch eine weitere Rektifikation (29) in einen im wesentlichen C$_{2+}$- bzw. C$_{3+}$-Kohlenwasserstoffe enthaltenden weiteren Produktstrom (30) und einen überwiegend leichter siedende Komponenten enthaltenden weiteren Restgasstrom (31) zerlegt wird.

11. Verfahren nach Anspruch 10, dadurch gekennzeichnet, daß der weitere Restgasstrom (31, 38) mindestens teilweise (41) arbeitsleistend entspannt (42, 43) wird und der Druck der (ersten) Rektifikation (7) zwischen dem erhöhten Druck des zu zerlegenden Gasstroms (1) und dem Druck des arbeitsleistend entspannten Restgasstroms (41) liegt.

12. Verfahren nach den Ansprüchen 6 und 11, dadurch gekennzeichnet, daß der nicht kondensierte Anteil (21) des Restgases (13) der (ersten) Rektifikation (7) dem arbeitsleistend entspannten Teil (44) des weiteren Restgases (31) zugemischt wird (51).

13. Anlage zur Durchführung des Verfahrens nach einem der Ansprüche 1 bis 12 mit mindestens einem Wäremetauscher (2) zur Abkühlung und partiellen Kondensation des Gasstroms (1), mit einem Abscheider (4) zur Abtrennung des partiell kondensierten Teils (5) des Gasstroms und mit einer Rektifiziersäule (7) zur Zerlegung des partiell kondensierten Teils des Gasstroms, gekennzeichnet durch eine Rückwaschkolonne (20), deren unterer Bereich mit dem Dampfraum des Abscheiders verbunden und deren oberer Bereich mit dem Kopf der Rektifiziersäule (7) verbunden ist, wobei zwischen dem Kopf der Rektifiziersäule und dem oberen Bereich der Rückwaschkolonne ein Wärmetauscher (15), ein weiterer Abscheider (17) und eine Pumpe (19) geschaltet sind.

14. Anlage nach Anspruch 13, dadurch gekennzeichnet, daß der Abscheider (4) und die Rückwaschkolonne (20) in einem gemeinsamen Behälter (4/20) angeordnet sind.

15. Anlage nach Anspruch 14, dadurch gekennzeichnet, daß die Rückwasclikolonne (20) oberhalb des Abscheiders (4) angeordnet und durch einen Kaminboden (22) vom Abscheider getrennt ist.

## Claims

1. Process for the separation of C$_{5+}$-hydrocarbons from a gas stream (1) containing light hydrocarbons and possibly components boiling more easily than methane, in which the gas stream (1) under increased pressure is cooled, partially condensed (2) and separated (4) into a liquid and a gaseous fraction and in which the liquid fraction is split up by rectification (7) into a product stream (8, 10) containing essentially C$_{5+}$-hydrocarbons and a residual gas stream (13) preponderantly containing easily boiling components, characterised in that the liquid fraction (5) separated after the partial condensation (2) is expanded (6) to a lower pressure before the rectification (7) and the gaseous fraction separated after the partial condensation is fed to a return flow washing column (20) in which C$_{5+}$-hydrocarbons are washed out of the gaseous fraction with the residual gas (13) obtained by the rectification after the partial condensation of the latter and the liquid fraction (24) accumulating in the sump of the return flow washing column (20) is fed after expansion to the rectification (7).

2. Process according to Claim 1, characterised in that the liquid fraction (24) accumulating in the sump of the return flow washing column (20) is fed to the rectification (7) as reflux liquid.

3. Process according to Claim 1 or 2, characterised in that the liquid fraction (5) obtained by partial condensation (2) is separated at a temperature between 10 and -15°C.

4. Process according to one of Claims 1 to 3, characterised in that the rectification (7) is so carried out that a part of the C$_3$-and C$_4$-hydrocarbons are withdrawn with the C$_{5+}$-product stream of the rectification (8, 10) and no C$_{5+}$-hydrocarbons are withdrawn with the residual gas stream (13).

5. Process according to one of Claims 1 to 4, characterised in that after the partial condensation (2, 15) of the residual gas (13) of the rectification (7), a phase separation (17) takes place and the liquid fraction (18) thereby obtained is pumped (19) to a higher pressure and fed to the return flow washing column (20).

6. Process according to Claim 5, characterised in that the uncondensed portion of the residual gas (21) from the rectification is withdrawn at low pressure as a residual gas stream (21, 51).

7. Process according to one of Claims 1 to 6, characterised in that the partial condensation (2, 15) of the residual gas (13) of the rectification (7) is effected by cooling to temperatures between -25 and -50°C.

8. Process according to one of Claims 1 to 7, characterised in that the residual gas (13) obtained by the rectification (7) is liquefied before its introduction into the return flow washing column (20) to 50 to 99 %, preferably to 70 to 95 %.

9. Process according to one of Claims 1 to 8, characterised in that before the rectification (7), the liquid fraction (5) is heated at least in part against the gas stream (1) to be cooled.

10. Process according to one of Claims 1 to 9, characterised in that the gaseous fraction (25) leaving the return flow washing column (20) is separated (27) by further cooling and partial condensation (15, 39) into a further liquid (28) and gaseous (35) fraction and the liquid fraction (28) so obtained is split up in a further rectification (29) into a further product stream (30) containing essentially $C_{2+}$- and/or $C_{3+}$-hydrocarbons and a further residual gas stream (31) containing preponderantly easily boiling components.

11. Process according to Claim 10, characterised in that the further residual gas stream (31, 38) is at least partially (41) expanded with the production of external work (42, 43) and the pressure of the (first) rectification (7) lies between the increased pressure of the gas stream (1) to be separated and the pressure of the residual gas stream (41) which has been expanded with the performance of external work.

12. Process according to Claim 6 and 11, characterised in that the uncondensed portion (21) of the residual gas (13) of the (first) rectification (7) is mixed (51) with the part (44) of the further residual gas (31) which has been expanded with the performance of external work.

13. Apparatus for carrying out the process of one of Claims 1 to 12, comprising at least one heat exchanger (2) for the cooling and partial condensation of the gas stream (1), a separator (4) for the separation of the partially condensed part (5) of the gas stream and a rectification column (7) for splitting up the partially condensed part of the gas stream, characterised by a return flow washing column (20) whose lower region is connected with the vapour space of the separator and whose upper region is connected with the head of the rectification column (7), wherein between the head of the rectification column and the upper region of the return flow washing column, a heat exchanger (15), a further separator (17) and a pump (19) are inserted.

14. Apparatus according to Claim 13, characterised in that the separator (4) and the return flow washing column (20) are arranged in a common container (4/20).

15. Apparatus according to Claim 14, characterised in that the return flow washing column (20) is arranged above the separator (4) and is separated from the separator by a chimney plate (22).

**Revendications**

1. Procédé de séparation des hydrocarbures en $C_{5+}$ contenus dans un courant gazeux comprenant des hydrocarbures légers et éventuellement des constituants à point d'ébullition plus bas que celui du méthane, dans lequel un courant gazeux (1) obtenu sous pression élevée est refroidi, partiellement condensé (2) et séparé (4) en deux fractions, l'une liquide et l'autre gazeuse, la fraction liquide étant séparée par rectification (7) en un courant de produit (8, 10) contenant principalement des hydrocarbures en $C_{5+}$ et un courant gazeux résiduel (13) contenant en majorité les fractions plus légères, caractérisé en ce que la fraction liquide (5) obtenue après condensation partielle (2), préalablement à la rectification (7) est soumise à une détente (6) à pression plus basse, en ce que les hydrocarbures en $C_{5+}$ sont retirés de la fraction gazeuse résultant de la condensation partielle, par lavage (20) à contre-courant, à l'aide d'une fraction préalablement condensée du gaz résiduel (13) provenant de la rectification, et en ce que la fraction liquide recueillie en cuve de la colonne de lavage (20) à contre-courant est, après détente, soumise à la rectification (7).

2. Procédé selon la revendication 1, caractérisé en ce que la fraction liquide produite (24) en cuve de la colonne de lavage à contre-courant (20) est chargée dans la rectification (7) en tant que liquide de reflux.

3. Procédé selon les revendications 1 ou 2, caractérisé en ce que la fraction liquide (5) conservée sous condensation partielle (2) est séparée (4) à une température comprise entre 10 et -15°C.

4. Procédé selon l'une des revendications 1 à 3, caractérisé en ce que la rectification (7) est effectuée de telle manière qu'une partie des hydrocarbures en $C_3$ et $C_4$ de la rectification (8, 10) soit retirée avec le courant de produit d'hydrocarbure en $C_{5+}$, cependant qu'aucun hydrocarbure en $C_{5+}$ n'est retiré avecle gaz résiduel (13).

5. Procédé selon l'une des revendications 1 à 4 caractérisé en ce qu'après la condensation partielle (2, 15) du gaz résiduel (13) et après la rectification (7), on opère une séparation de phases (17), la fraction liquide obtenue (18) étant pompée sous haute pression (19) pour alimenter la colonne de lavage à contre-courant (20).

6. Procédé selon la revendication 5, caractérisé en ce que la partie non condensée du gaz résiduel de la rectification (21) est retirée à basse pression comme courant de gaz résiduel (21, 51).

7. Procédé selon l'une des revendications 1 à 6, caractérisé en ce que la condensation partielle (2, 15) du gaz résiduel après rectification (7) s'effectue par refroidissement à des températures allant de -25°C à -50°C.

8. Procédé selon l'une des revendications 1 à 7, caractérisé en ce que le gaz résiduel (13) obtenu après rectification (7) est liquéfiée de 50 à 99 %, de préférence de 70 à 95 % avant d'alimenter la colonne de lavage à contre-courant (20).

9. Procédé selon l'une des revendications 1 à 8, caractérisé en ce que la fraction liquide (5) est chauffée au moins partiellement avant la rectification (7), par échange thermique avec le courant de gaz (1) à refroidir.

10. Procédé selon l'une des revendications 1 à 9, caractérisé en ce que la fraction gazeuse (25) issue de la colonne de lavage à contre-courant (20) est separee (27) en deux autres fractions l'une liquide (28) l'autre gazeuse (35), par refroidissement supplémentaire et condensation partielle (15, 39) et en ce que la fraction liquide ainsi obtenue est fractionnée (29) en un premier courant de produit (30) contenant principalement des hydrocarbures en $C_{2+}$ (resp. $C_{3+}$) et aussi en un autre courant de gaz résiduel (31) contenant en majorité des composés à plus bas point d'ébullition.

11. Procédé selon la revendication 10, caractérisé en ce que le courant de gaz résiduel (31, 38) est détendu (42, 43) avec production de travail et en ce que la pression lors de la (première) rectification (7) est située entre la pression élevée du courant de gaz à fractionner (1) et la pression du courant de gaz résiduel après détente avec production de travail (41).

12. Procédé selon les revendications 6 et 11, caractérisé en ce que la partie non condensée (21) du gaz résiduel (13) de la (première) rectification (7) est mélangé (51) à la partie (44) de l'autre gaz (31) détendue avec production de travail.

13. Installation pour la mise en oeuvre du procédé selon l'une des revendications 1 à 12, comportant au moins un échangeur de chaleur (2) pour le refroidissement et la condensation partielle du courant de gaz (1), un séparateur (4) pour la séparation de la partie condensée partiellement (5) du courant de gaz, ainsi qu'une colonne de rectification (7) pour le fractionnement de la partie partiellement condensée du courant de gaz, caractérisée par une colonne de lavage à contre-courant (20) dont la cuve est reliée à l'enceinte de vapeur du séparateur et dont la tête est reliée à la tête de la colonne de rectification (7), cependant qu'un échangeur de chaleur (15), un séparateur supplémentaire (17) et une pompe (19) sont branchés entre la tête de la colonne de rectification et le haut de la colonne de lavage à contre-courant.

14. Installation suivant la revendication 13, caractérisée en ce que le séparateur (4) et la colonne de lavage à contre-courant (20) sont aménagés dans un méme récipient (4/ 20).

15. Installation selon la revendication 14, caractérisée en ce que la colonne de lavage à contre-courant (20) est installée au dessus du séparateur (4) dont elle est séparée par un plateau à cheminée (22).

Fig.1

Fig.2